# EUROPÄISCHE PATENTANMELDUNG

(11) **EP 1 121 934 A1**
(43) Veröffentlichungstag der Anmeldung: **08.08.2001**
(21) Anmeldenummer: 01108684.0
(22) Anmeldetag: 03.02.1997
(51) Int. Cl.: A61K 33/00, A61P 9/10

(54) **Neue Verwendung von Stickstoffmonoxid**

(30) Priorität: 07.02.1996 DE 19604361
(62) Teilanmeldung aus: 97904375.9
(71) Anmelder: SCHWARZ PHARMA AG, D-40789 Monheim/Rhld. (DE)
(72) Erfinder: Kelm, Malte, Prof. Dr., 40225 Düsseldorf (DE)

(57) **Zusammenfassung**

Die Erfindung betrifft die Verwendung von Stickstoffmonoxid (NO) zur Herstellung eines Arzneimittels zur intravaskulären Verabreichung bei Säugern.

Mit der erfindungsgemäßen NO-Lösung kann NO lokal in hohen Dosen in Gefäßen (z.B. Koronargefäß) gezielte lokale biologische Effekte auslösen, wie Relaxation der glatten Gefäßmuskulatur, Inhibition der Adhäsion von Thrombozyten etc., ohne daß es aufgrund hoher Dosen zu systemischen Begleiterscheinungen, wie z.B. einem kritischen Druckabfall kommt.

## Beschreibung

Die vorliegende Erfindung betrifft die Verwendung von Stickstoffmonoxid (NO) zur Herstellung eines Arzneimittels zur intravaskulären Verabreichung bei Säugern.

Physiologisch präsentes Stickstoffmonoxid (NO) kann durch verschiedene biologische Funktionen wesentliche Schlüsselprozesse in der Entwicklung der Arteriosklerose beeinflussen, wie die Relaxation der glatten Gefäßmuskulatur, die Hemmung der Adhäsion von Thrombozyten, Granulozyten und Monozyten an der Gefäßwand, die Inhibition der Proliferation sekretorischer glatter Muskelzellen und die direkte Beeinflussung des endothelialen Stoffwechsels.

So konnte in den letzten Jahren gezeigt werden, daß endotheliales Stickstoffmonoxid (NO) über eine Vielzahl von Mechanismen sowohl den Stoffwechsel als auch die Funktion der Gefäßwand beeinflußt:
1. es führt über Stimulation der löslichen Guanylatzyklase zu einer Relaxation der glatten Gefäßmuskulatur, und
2. es ist somit an einer Regulation des peripher vaskulären Widerstandes und arteriellen Blutdruckes beteiligt,
3. ferner hemmt es die Mitogenese und Proliferation der glatten Gefäßmuskelzellen,
4. es unterdrückt die Adhäsion von Thrombozyten, Monozyten und neutrophilen Granulozyten an der Gefäßwand, und
5. es beeinflußt direkt den Stoffwechsel der Endothelzellen, z.B. durch eine veränderte Expression von Adhäsionsmolekülen an der Oberflächenmembran. Übersichten hierzu finden sich bei
1. Moncada, S. and Higgs, A. The L-arginine-nitric oxide pathway. N. Engl. J. Med. 329: 2002-2012, 1993.
2. Radomski, M.W. and Moncada, S. Regulation of vascular homeostasis by nitric oxide. Thromb. Haemost. 70: 36-41, 1993.
3. Snyder, S.H. and Bredt, D.S. Biological roles of nitric oxide. Sci AM 266 (5): 68-77, 1992.
4. Gibbons, G.H. and Dzau, V.J. The emerging concept of vascular remodeling. N. Engl. J Med. 330: 1431-1438, 1994.

Sämtliche der genannten Prozesse stellen Schlüsselereignisse in der Entwicklung pathologischer Gefäßwandveränderungen bei der Arteriosklerose dar. Anerkannte Risikofaktoren der Arteriosklerose, welche auch die endotheliale Funktion beeinflussen, sind: die arterielle Hypertonie, die Hyperlipoproteinämie, der Diabetes mellitus, ein Nikotinkonsum und möglicherweise das Alter und Geschlecht (siehe: Moncada, S. and Higgs, A. The L-arginine-nitric oxide pathway. N. Engl. J. Med. 329: 2002-2012, 1993; Ross, R. The pathogenesis of atherosclerosis - an update. N. Engl. J. Med. 324 (No 8): 488-500, 1986; Safar, M.E. and Frohlich, E.D. The arterial system in hypertension. A prospective view. Hypertension 26: 10-14, 1995).

Der pathophysiologisch relevante Nachweis einer geänderten Aktivität der endothelialen NO-Synthese gelang bis jetzt jedoch nur in experimentellen Ansätzen mit kultivierten Zellen oder isolierten Organkreisläufen, in denen eine Quantifizierung von NO bzw. seiner Abbauprodukte möglich war. Am Menschen konnten diesbezüglich bisher jedoch nur phänomenologische Befunde hinsichtlich einer veränderten Acetylcholin-induzierten Flußantowrt in verschiedenen Stromgebieten beschrieben werden, als indirekter Marker einer Endothel-abhängigen Vasodilatation. Eine exakte Quantifizierung der Aktivität der endothelialen konstitutiven NO-Synthase am Menschen als Indikator einer endothelialen Dysfunktion war bisher nicht möglich.

Schließlich wurden in den vergangenen Jahrzehnten verschiedene Klassen von NO-Donoren zur Therapie arteriosklerotischer Gefäßwandveränderungen wie zum Beispiel der koronaren Herzerkrankung erfolgreich entwickelt und eingesetzt (Übersichten hierzu finden sich bei: Feelisch, M. The biochemical pathways of nitric oxide formation from nitrovaso-dilators: appropriate choice of exogenous NO donors and aspects of preparation and handling of aqueous NO solutions. J. Cardiovasc. Pharmacol. 17 (suppl. 3): S25-S33, 1991; Feelisch M. and Noack, E. The vitro metabolism of nitrovasodilators and their conversion into vasoactive species. In: Heart Failure Mechanisms and Management, edited by Lewis, B.S. and Kimchi, A., Springer: Berlin, Heidelberg, 1991, p.241-255; Harrison, D.G. and Bates, J.N. The nitrovasodilators. Circulation 87: 1461-1467, 1993; De Caterina, R. Nitrate als Thrombozytenfunktionshemmer. Z. Kardiol. 83: 463-473, 1994).

Die NO-Donoren wirken sowohl im arteriellen als auch im venösen Teil des Kreislaufes und beeinflussen NO-abhängig auch die Interaktion der Gefäßwand mit den korpuskulären Bestandteilen des Blutes (Thrombozyten, neutrophile Granulozyten und Monozyten). Bis heute gibt es jedoch noch keine therapeutische Entwicklung, die es erlaubt, NO direkt in Form von authentischen NO-Lösungen am menschlichen Kreislauf einzusetzen. Dies mag in der äußerst raschen Metabolisierung und Inaktivierung von NO im menschlichen Kreislauf begründet sein, was einerseits die erfolgreiche und zielgerechte Einbringung von NO in den menschlichen Kreislauf behindert, aber andererseits den Vorteil bietet, potential lokal sehr hohe NO-Dosen erzielen zu können, ohne systemisch begleitende Nebenwirkungen in Kauf nehmen zu müssen. Insbesondere letzterer Punkt erscheint im Hinblick auf die o.g. umfassenden antiarteriosklerotischen Eigenschaften von NO als äußerst relevant für sämtliche intravaskulären Interventionen an peripheren und koronaren Arterien.

In den Industriestaaten mit hoher Zivilisation wie USA und Deutschland werden derzeit jährlich über 370.000 koronare Interventionen (PTCA etc.) durchgeführt (siehe Gleichmann, U., Mannebach, H., and Lichtlen, P. 10. Bericht über Struktur und Leistungszahlen der Herzkatheterlabors in der Bundesrepublik Deutschland. Z Kardiol 84:327-333, 1995).

Die initiale Erfolgsrate beträgt bis zu 90 %, in 10 % der PTCAs kommt es jedoch zu einem frühzeitigen Verschluß bzw. zu einer Trombusbildung. Darüber hinaus kommt es in 25 - 50 %, je nach Literaturangabe, zur Ausbildung einer Restenose in den ersten sechs Monaten nach dem Eingriff. Die Gesamtzahl aller weltweit durchgeführten Angioplastien wird für das Jahr 2000 mit über einer Million veranschlagt, was wiederum bedeutet, daß mit bis zu 400.000 Restenosen jährlich zu rechnen ist. Diese Zahlen unterstreichen den dringenden Handlungsbedarf zur Senkung der akuten und langfristigen Restenoserate nach PTCA. Systemische pharmakologische Interventionsstudien mit Kalziumantagonisten, organischen Nitraten und ACE-Hemmern zeigten bis jetzt keinen Einfluß auf die Restenoserate. Auch die Entwicklung verschiedener mechanischer Alternativverfahren (direktionale Atherektomie, Laserangioplastie, Stent-Implantation, Rotablation) brachten keine allumfassende Senkung der Restenoserate, sondern blieben auf bestimmte Nischenindikationen begrenzt. Dies führte in letzter Zeit zur Entwicklung neuer LDD-Systeme (local drug delivery), wie beschichtete Stentprothesen, dem Kaplan-Simpson-Infusionskatheter (Stanford University), dem Mikroinfusionskatheter (Cordis) oder dem porösen PTCA-Ballonkatheter (ACS). Mit diesen Systemen erscheint es erstmals möglich, lokal hohe Dosen eines Pharmakons in die Gefäßwand der Koronararterien einzubringen. Aufgrund des raschen Metabolismus von NO im menschlichen Blut, erscheint es nun als äußerst wünschenswert und sinnvoll, NO lokal in hohen Dosen während koronarer Interventionsmaßnahmen in das Koronargefäß einzubringen und somit an lokaler Stelle alle einleitend genannten biologischen Effekte von NO, wie Relaxation der glatten Gefäßmuskulatur, Inhibition der Adhäsion von Thrombozyten, neutrophilen Monozyten zu nutzen, ohne daß es aufgrund hoher Dosen zu systemischen Begleiterscheinungen, wie z.B. einem kritischen Druckabfall kommt.

Wie bereits einleitend erwähnt, stellen die arterielle Hypertonie, die Hyperlipoproteinämie und der Diabetes mellitus die wesentlichen atherogenen Risikofaktoren dar. Allein für die arterielle Hypertonie und ihre kardiovaskulären Folgeerkrankungen weisen epidemiologische Daten eine Gesamtletalität von 25 % auf; (siehe Strauer, B.E. Das Hochdruckherz, Berlin, Heidelberg, New York: Springer Verlag, 1991. pp. 1-241).

Eine endotheliale Dysfunktion, die als wesentlicher Schrittmacher einer Arteriosklerose definiert werden konnte, ist bis zum heutigen Zeitpunkt nicht durch einen klinischen Routineparameter erfaßbar. Unter dem Gesamtaspekt einer frühzeitigen Diagnose als auch einer differentialtherapeutischen Beeinflussung einer solchen endothelialen Dysfunktion ist somit ein zuverlässiges Detektionsverfahren für den Bereich der kardiovaskulären Medizin wünschenswert.

Aufgabe der vorliegenden Erfindung ist es daher, in den Gefäßen von Säugern NO lokal in hohen Dosen zur Verfügung zu stellen.

Diese Aufgabe wurde, wie nachstehend erläutert und wie sich aus den Patentansprüchen ergibt, gelöst.

Eine Ausführungsform der Erfindung ist eine pharmazeutische Zusammensetzung einer sterilen, injizierbaren physiologisch annehmbaren Lösung mit einem Gehalt an authentischem NO.

Nach einer Ausführungsform der Erfindung besteht die pharmazeutische Zusammensetzung aus 0,9 %-iger mit NO gesättigter physiologischer Natriumchlorid-Lösung.

Bei der erfindungsgemäßen pharmazeutischen Zusammensetzung nimmt die Sättigungskonzentration des NO in der 0,9 %-igen Natriumchloridlösung mit zunehmender Temperatur differentiell ab. Die Werte der Sättigungskonzentration mit NO der 0,9 %-igen Natriumchlorid-Lösung betragen bei 0°C 3,26 NO [µmol/l], bei 25°C 1,85 NO [µmol/l], bei 37°C 1,52 NO [µmol/l] und bei 50°C 1,26 NO [µmol/l].

Die pharmazeutische Zusammensetzung wird erfindungsgemäß hergestellt, indem unter Sauerstoffausschluß Argon durch eine sterile, injizierbare 0,9 %-ige Natriumchloridlösung geleitet wird, bis jeglicher Restsauerstoffgehalt entfernt ist und anschließend bis zur Sättigungskonzentration des NO in der 0,9 %-igen Natriumchloridlösung NO durch diese geleitet wird.

Die erfindungsgemäße NO-Lösung kann nach einer weiteren Ausführungsform der Erfindung zur Herstellung einer pharmazeutischen Zusammensetzung zur Behandlung der endothelialen Dysfunktion bei Säugern verwendet werden.

Das nachfolgende Ausführungsbeispiel 1 erläutert den Aufbau der Vorrichtung zur Herstellung einer sterilen, injizierbaren NO-Lösung und ihre Herstellung.

### Ausführungsbeispiel 1

a) Konfiguration der Vorrichtung
   Die Konfiguration der Vorrichtung ist in der Zeichnung 1 wiedergegeben. In ihr bedeuten:
   1 NO 3,0-Reservoir (AGA);
   2 Argon 5,0-Reservoir (Linde);
   3 Gaswaschflasche mit Pyrogallol 5 % (Pyrogallol z.A. 612, Merck) in gesättigter KOH (w:v.);
   4 Gaswaschflasche mit KOH z.A. 5033, (Merck), 20 %;
   5 Gaswaschflasche, trocken;
   6 Dreihalsrundkolben (250 ml), beladen mit 0,9 %-iger NaCI-Lösung mit 3 Schliffeinsätzen, in denen jeweils eingebaut sind:
      a) Abschlußhahn
      b) Luer-Lock 3-Wegehahn
      c) 3-Wegehahn
   7 Gaswaschflasche, leer, als Wasserfalle;
   8 Gaswaschflasche mit KOH z.A. 5033 (Merck), als Wasserfalle;

   A kennzeichnet die jeweiligen gasdichten Glasschliffe;
   B gasdichter Dreiwegehahn;
   C kennzeichnet die gasdichten Glasschliffe;
   D gasdichter Dreiwegehahn;
   E gasdichte Glasschliffe zur Verbindung der jeweiligen Gaswaschflaschen

   Alle Geräteteile sind mit gasdichten Schläuchen (Typ Tygon R 3603) verbunden. Als Dichtungen werden Teflondichtungen Plastibrand (Brand) und als Filter solche vom Typ Millex GS, 0,2 µm (Millipore) verwendet.
b) Herstellung einer sterilen NO-Lösung
   Sämtliche Glasgeräte werden mittels konzentrierter Natronlauge (NaOH) gereinigt und jeweils 10 mal mit Aqua bidestillata und HPLC-Wasser nachgespült. Dann werden die nacheinander geschalteten Gaswaschflaschen (3), (4), (5), von denen in (3) Pyrogallol (5 %) in gesättigter KOH (w:v) zur Entfernung von Sauerstoffspuren, in (4) 20 %-ige KOH zur Entfernung höherer Stickoxide vorgelegt sind und (5) trocken ist, und der damit verbundene Dreihalsrundkolben in dem NaCI-Lösung (0,9 %) vorgelegt ist, für 45 min. mit Argon begast.
   Der innerhalb des Steigrohres befindliche Teil der NaCI-Lösung wird verworfen. Aus einer sterilen, gasdichten Spritze und den zugehörigen Leitungssystemen sowie Filtern wird der Sauerstoff durch einen sterilen 0,2 µm Filter mittels Argon entfernt und das für die Verdünnung der NO-Lösung notwendige NaCI-Volumen in die sauerstofffreie, gasdichte Spritze abgenommen. Schließlich wird die Anlage für 45 min. mit Stickstoffmonoxid (NO) begast.
   Aus den Filtern und Leitungssystemen, die zu der Spritze mit der vorgelegten Verdünnungslösung führen, wird der Sauerstoff erneut mit Argon vollständig entfernt. Der innerhalb des Steigrohres befindliche Teil der NO-Lösung wird verworfen und danach das gesamte Leitungssystem und der sterile Filter mit NO-Lösung durchgespült. Das an NO-Lösung nötige Volumen wird sofort in das mit vorgelegter NaCI-Lösung vorbereitete gasdichte Primärpackmittel über den Dreiwegehahn (B) durch sterile Filter übernommen, das jeweils eine Ampulle, Vial, Spritze etc. sein kann.

Für eine optimale Behandlung und Einstellung des Patienten ist auch ein Detektionsverfahren zur quantitativen Erfassung einer endothelialen Dysfunktion an Säugern wünschenswert.

Denn bisher konnte eine endotheliale Dysfunktion bei Patienen mit arterieller Hypertonie, Diabetes mellitus und/oder Hyperlipoproteinämie nur durch aufwendig diagnostische Maßnahmen wie eine arterielle Punktion mit nachfolgender lokaler Applikation von Acetylcholin und hämodynamischer Quantifizierung der daraus folgenden Flußantwort im untersuchten Stromgebiet im Vergleich zu einem altersentsprechenden Normalkollektiv gesichert werden; (siehe Calver, A., Collier, J., and Vallance, P. Inhibition and stimulation of nitric oxide synthesis in the human forearm arterial bed of patients with insulin-dependent diabetes. J.Clin.Invest. 90:2548-2554, 1992; Creager, M.A., Cooke, J.P., Mendelsohn, M.E., Gallagher, S.J., Coleman, S.M., Loscalzo, J., and Dzau, V.J. Impaired vasodilation of forearm resistance vessels in hypercholesterolemic humans. J.Clin.Invest. 86:228-234, 1990; Kelm, M., Preik, M., Motz, W., and Strauer, B.E. Endotheliale Funktion bei Patienten mit arterieller Hypertonie. In: Zelluläre Mechanismen der Herz-Kreislaufregulation, edited by Ganten, D., Stuttgart: Schattauer, 1993, p. 139-150; Linder, L., Kiowski, W., Bühler, F.R., and Lüscher, T.F. Indirect evidence for release of endothelium-derived relaxing factor in human forearm circulation in vivo. Circulation 81: 1762-1767, 1990; Panza, J.A., Epstein, S.E., and Quyyumi, A.A. Circadian variation in vascular tone and its relation to a a-sympathetic vasoconstrictor activity. N.Engl.J.Med 325: 986-990, 1991).

Ferner wurde versucht, in Longitudinalstudien über diesen rein phänomenologischen Ansatz eine mögliche therapeutische Beeinflussung einer endothelialen Dysfunktion z.B. bei Patienten mit arterieller Hypertonie durch eine blutdrucksenkende Therapie mit ACE-Hemmern zu dokumentieren, was jedoch aufgrund der großen methodischen Streubreite des Untersuchungsansatzes nicht gelang; (siehe Creager, M.A. and Roddy, M.-A. Effect of captopril and enalapril on endothelial function in hypertensive patients. Hypertension 24: 499-505, 1994).

Ferner kann über den rein phänomenologischen Ansatz einer geänderten Acetylcholinabhängigen Flußantwort nicht differenziert werden, inwieweit einer arteriosklerotisch bedingten endothelialen Dysfunktion eine verminderte NO-Synthese und/oder ein vermehrter NO-Abbau zugrunde liegt; (siehe Keim, M., Preik, M., Köster, A., Heinzelmann, A., Motz, W., and Strauer, B.E. Reduced nitric oxide dependent vasodilation following application of organic nitrates in essential hypertension. In: The biology of nitric oxide, edited by Moncada, S., London: Portland Press Ltd., 1994, p. 509-513; Kelm, M., Feelisch M., Krebber, T., Deussen, A., Motz, W., and Strauer, B.E. The role of nitric oxide (NO) in the regulation of coronary vascular tone in hearts from hypertensive rats: maintenance of NO forming capacity and increased basal production of NO. Hypertension, 25: 186-193, 1995; Kelm, M., Preik, M., Hafner, D., Strauer, B.E. Evidence for a multifactorial process involved in the impaired flow responses to nitric oxide in hypertensive patients with endothelial dysfunction. Hypertension, im Druck 1996).

Das nachfolgend dargestellte Detektionsverfahren erlaubt die Messung des Gehalts von Nitrit im Vollblut von Säugern, insbesondere im menschlichen Vollblut, wobei Nitrit der spezifische Marker der Aktivität der endothelialen konstitutiven NO-Synthese im menschlichen Kreislauf ist.

Das nachfolgend genannte Ausführungsbeispiel 2 erläutert das Detektionsverfahren.

### Ausführungsbeispiel 2

### Verfahren zur Nitritbestimmung in humanem Vollblut

1 ml Blut wird in eine Spritze mit 1 ml vorgelegter Stopplösung aus 1 mol/L konzentrierter NaOH verbracht und unmittelbar anschließend 50 µl einer 1 mol/L konzentrierten Phosphorsäure (H₃PO₄) zur Neutralisation des erhaltenen Blut/NaOH-Gemisches zugegeben. Dieses Gemisch wird 5 min. mit 10.000 g in einer Zentrifuge (Universal 30RF, Fa. Hettich) zentrifugiert. Der erhaltene Überstand wird in Ultrafiltrationsröhrchen (Sartorius cut-off 10.000) dekantiert. Es wird erneut 15 min. mit 2.000 g (Eppendorfzentrifuge 5402 C, Fa. Eppendorf) zentrifugiert. Das erhaltene wasserklare Ultrafiltrat wird in 1,5 ml Volumen fassende Reaktionsgefäße übernommen und mit HPLC-Wasser im Verhältnis 1:10 verdünnt.

Daran schließt sich entweder unmittelbar die Analyse an oder die erhaltenen Proben werden bei -80°C für die Dauer eines Monats gelagert.

### Pharmakologische Untersuchungen

### 1. Nitrit als spezifisches Diagnostikum der cNOS

Es wurden die eigenen Erkenntnisse zum Metabolismus von NO im menschlichen Kreislauf zugrunde gelegt und darauf aufbauend Nitrit als spezifischer Marker der Aktivität der endothelialen konstitutiven NO-Syntase exakt und valide vermessen.

Es wurde die im Ausführungsbeispiel 2 genannte spezifische Stopplösung eingesetzt, die es ermöglicht, die rasche Konversion von Nitrit zu Nitrat im menschlichen Blut zu unterbinden, ohne daß es dadurch während der Probenaufarbeitung zu einem wesentlichen Verlust von Nitrit kommt und die Recovery für Nitrit nach Aufarbeitung und anschließender Vermessung der Probe gegeben war. Ferner wurde nachfolgend eine hochsensitive Meßmethodik entwickelt, welche mittels kombinierter ultravioletter und elektrochemischer Detektion, integriert in einer 2 Kanal-HPLC-Anlage, die simultane und hochsensitive Quantifizierung von Nitrit und Nitrat in Proben menschlichen Vollblutes bzw. des von Säugern ermöglicht. Folgende Resultate wurden mit Probanden erzielt:

In Übereinstimmung mit experimentellen Studien, welche eine unterschiedliche NO-Produktion in arteriellen und venösen Endothelzellen unter Basalbedingungen zeigten, konnte am Menschen in arteriellen Blutproben eine höhere Nitritkonzentration (1310±50 nmol/l) im Vergleich zu Proben aus venösen Stromgebieten (1100 ±40 nmol/l) nachgewiesen werden. Im Gegensatz dazu zeigten, wie bereits oben angesprochen, die Serumwerte für Nitrat keine spezifischen Unterschiede und betrugen im Mittel 42 ± 6 µmol/l sowohl in Proben aus dem arteriellen als auch dem venösen Teil des menschlichen Kreislaufes (n=5). Darüber hinaus konnte am Modell der Unterarmzirkulation des Menschen gezeigt werden, daß lokale Infusion der Endothel-abhängigen Dilatatoren Acetylcholin und Bradykinin in die A. brachialis dosisabhängig zu einem mehr als vierfachen Anstieg des Ruheflusses führt (n=7). Während dieses Flußanstieges konnte dosisabhängig für beide Mediatoren spezifisch ein Anstieg der Serumkonzentration an Nitrit in Proben aus der Vena cubitalis dokumentiert werden. Somit kam es trotz eines mehr als vierfachen Anstieges des Blutflusses in dem Stromgebiet des Unterarmes zu einer spezifischen Erhöhung der Serumkonzentration an Nitrit nach Infusion beider Stimulatoren der konstitutiven endothelialen NO-Synthase. Dies bedeutet, daß Nitrit spezifisch und dosisabhängig eine gesteigerte endotheliale NO-Produktion in diesem Stromgebiet widerspiegelt. Darüber hinaus konnte für beide Substanzen eine hochsignifikante Korrelation zwischen dem Flußanstieg und dem Anstieg der Konzentration an Nitrit im Serum als auch der Freisetzungsrate über das Unterarmstromgebiet nachgewiesen werden (siehe Zeichnung 2 und 3). Außerdem konnte in Kontrollversuchen gezeigt werden, daß der Endothel-unabhängige Vasodilatator Papaverin zu einem vergleichbaren Anstieg des Blutflusses um den Faktor 4 führte, ohne daß es nachweislich zu einem Anstieg der Serumkonzentration an Nitrit kam. Ferner konnte durch zeitgleiche Infusion von L-NMMA als stereospezifischem Inhibitor der konstitutiven endothelialen NO-Synthase (6 µmol/min) eine 40 %ige Reduktion der Flußantwort und der Serumkonzentration an Nitrit im Vergleich zu Kontrollbedingungen (n=3) nachgewiesen werden. Zusammenfassend belegen diese Untersuchungen, daß es mit der entwickelten Methodik erstmalig möglich ist, lokal und spezifisch die Aktivität der endothelialen konstitutiven NO-Synthase im Gefäßsystem zu quantifizieren und somit ihren Funktionszustand im Hinblick auf eine endotheliale Dysfunktion zu diagnostizieren.

### 2. Therapie mit erfindungsgemäßen authentischen NO-Lösungen im Kreislauf von Säugern, speziell im menschlichen Kreislauf

Aufgrund des raschen Metabolismus von NO im menschlichen Kreislauf können die erfindungsgemäßen authentischen NO-Lösungen als therapeutisches Mittel eingesetzt werden, um lokal sehr hohe Wirkungskonzentrationen an NO erzielen zu können, ohne zeitgleiche delitäre systemische Nebenwirkungen auszulösen.

Es wurde das Modell der Unterarmzirkulation gewählt und wäßrige NO-Standards in aufsteigenden Dosierungen Probanden lokal appliziert. Aufgrund des Metabolismus von NO im menschlichen Blut eignet sich hierfür nicht eine kontinuierliche Infusion, sondern wesentlich effektiver ist die Bolus-Technik. Die Konzentrationen der gewählten Stammlösung und die Kinetik der Applikation müssen so gewählt werden, daß ein klar umschriebener Bolus der wäßrigen NO-Lösung, entsprechend dem zirkulären Kreislaufvolumen und der Transitzeit von der Applikationsstelle bis zu den Widerstandsarterien, reproduzierbar das anvisierte Stromgebiet erreicht. Wie in Zeichnungen 4 und 5 zu sehen ist, konnte mittels dieser Bolus-Technik NO reproduzierbar zu einem dosisabhängigen Anstieg des Blutflusses sowohl im Maximum als auch über die Fläche unter der Kurve der Flußantwort führen, ohne daß es systemisch nachweisbar zu einem Abfall des Blutdruckes kam. Ferner konnte in zusätzlichen toxikologischen Untersuchungen gezeigt werden, daß es in den verwandten Dosen zu keiner berichteten Veränderung des Methämoglobingehaltes im Blut der Probanden unter der NO-Applikation während der Untersuchung gekommen war. Zusätzlich konnte noch unter Anwendung der oben beschriebenen Stopplösung gezeigt werden, daß es unter der NO-Applikation in diesem regionalen Stromgebiet zu einem Anstieg der Serumnitritkonzentration in der Vena cubitalis um 111 ± 12 % kam, während sich die Serumkonzentration für Nitrat (45 ± 8 µmol/l) nicht signifikant unter der Intervention änderte. Die lokale Flußantwort auf diesen in Bolus-Technik applizierten wäßrigen NO-Standard korrelierte hochsignifikant mit der Körperoberfläche der einzelnen Probanden und war unabhängig von der Höhe der Herzfrequenz oder des arteriellen systemischen Blutdruckes.

### 3. Erläuterungen zu den Zeichnungen 2 bis 5

### Zeichnung 2

Beziehung zwischen Änderungen des Blutflusses im Unterarm (FBF) und der Serumkonzentration an Nitrit (A) sowie der Freisetzungsrate an NO bzw. Nitrit in die Unterarmzirkulation nach lokaler intraarterieller Infusion aufsteigender Dosen von Acetylcholin (Daten aus Abb. 1). Die lineare Regressionsanalyse ergab ein r = 0,997 und ein P = 0,003.

### Zeichnung 3

Beziehung zwischen Änderungen des Blutflusses im Unterarm (FBF) und der Serumkonzentration an Nitrit (A) sowie der Freisetzungsrate an NO bzw. Nitrit in die Unterarmzirkulation nach lokaler intraarterieller Infusion aufsteigender Dosen von Bradykinin (Daten aus Abb. 2). Die lineare Regressionsanalyse ergab ein r = 0,985 und ein P = 0,001.

### Zeichnung 4

Dosisantwortkurve für die NO-induzierte Vasodilatation in der Unterarmzirkulation. Aufgetragen ist der plethysmographisch bestimmte Anstieg des Blutflusses im Unteram (FBF) gegen die Dosis an NO, welche in Bolus-Technik aus salinen Standards von NO-Lösungen appliziert wurde (n=6, Mittelwert ± SEM).

### Zeichung 5

Dosisantwortkurve für die NO-induzierte Vasodilatation in der Unterarmzirkulation. Aufgetragen ist die Fläche unter der Kurve für die Änderungen des FBF, gleiche Probanden wie in Zeichnung 4/5 (n= 6, Mittelwert ± SEM).

## Patentansprüche

1. Verwendung von Stickstoffmonoxid (NO) zur Herstellung eines Arzneimittels zur intravaskulären Verabreichung zur Behandlung der endothelialen Dysfunktion bei Säugern.

2. Verwendung von Stickstoffmonoxid (NO) zur Herstellung eines Arzneimittels zur intravaskulären Verabreichung zur adjuvanten Behandlung intravaskulärer Interventionen bei Säugern.

3. Verwendung von Stickstoffmonoxid (NO) gemäß Anspruch 2, dadurch gekennzeichnet, daß die intravaskuläre Intervention an den Koronarien erfolgt.

4. Verwendung von Stickstoffmonoxid (NO) gemäß Anspruch 3, dadurch gekennzeichnet, daß die intravaskuläre Intervention eine perkutane transluminale coronare Angioplastie (PTCA) ist.
